# FASCICULE DE BREVET EUROPEEN

(11) **EP 2 350 108 B1**
(45) Date de publication et mention de la délivrance du brevet: **30.04.2014**
(21) Numéro de dépôt: 09749118.7
(22) Date de dépôt: 06.11.2009
(51) Int. Cl.: C07H 1/00, C07H 7/00

(54) **COMPOSES C-GLYCOSIDES ET PROCEDES DE PREPARATION DE COMPOSES C-GLYCOSIDES**
C-GLYCOSIDVERBINDUNGEN UND VERFAHREN ZUR HERSTELLUNG VON C-GLYCOSIDVERBINDUNGEN
C-GLYCOSIDE COMPOUNDS, AND METHOD FOR PREPARING C-GLYCOSIDE COMPOUNDS

(30) Priorité: 07.11.2008 FR 0857576; 26.11.2008 FR 0858024
(43) Date de publication de la demande: 03.08.2011
(73) Titulaire: Ecole Nationale Superieure de Chimie de Rennes, 35700 Rennes Cedex (FR); Armor Proteines, 50890 Conde-sur-Vire (FR)
(72) Inventeur: BENVEGNU, Thierry, F-35700 Rennes (FR); LEMIEGRE, Loïc, F-35700 Rennes (FR); RANOUX, Adeline, F-63000 Clermont-Ferrand (FR)
(74) Mandataire: Larcher, Dominique
(86) Numéro de dépôt international: PCT/EP2009/064792
(87) Numéro de publication internationale: WO 2010/052314

(56) Documents cités:
- WO-A-02/051828
- AUCAGNE V ET AL: "Synthetic Approaches to C-Glucosinolates" TETRAHEDRON, ELSEVIER SCIENCE PUBLISHERS, AMSTERDAM, NL, vol. 56, no. 17, 1 avril 2000 (2000-04-01) , pages 2647-2654, XP004197461 ISSN: 0040-4020

## Description

Le domaine de l'invention est celui de la synthèse des composés *C*-glycosides. De tels composés sont utilisés notamment pour la réalisation de compositions présentant des propriétés tensio-actives et pour la réalisation de compositions cosmétiques.

Plus précisément, l'invention concerne de tels composés *C*-glycosides ainsi que différents procédés de préparation de tels composés.

L'industrie des produits chimiques est à la recherche de composés respectueux de l'environnement, notamment en étant biodégradables et éco-compatibles, ainsi que de procédés de synthèse de tels composés répondant au mieux aux dispositions légales et réglementaires en matière d'écologie.

Ainsi, les composés tensioactifs à base de sucre présentent de nombreux avantages. En particulier, ils sont renouvelables, biodégradables, faiblement toxiques et apportent des propriétés originales notamment par rapport aux substrats d'origine pétrochimique. Parmi ces propriétés originales, on peut citer le très faible potentiel d'irritation de la peau et des propriétés cristallines liquides qui apportent des propriétés sensorielles recherchées en cosmétique.

Des composés à base de sucres tels que les alkylpolyglucosides (APG), les esters de sorbitan, les sucroesters ont été développés. Même si ces composés présentent un intérêt évident pour de nombreuses applications, la recherche de nouvelles molécules à base de sucre reste un domaine de recherche indispensable pour améliorer les performances des tensioactifs.

La synthèse de tensioactifs à partir de sucres est connue par les voies de l'estérification, de l'éthérification, de la glycosidation ou par la synthèse enzymatique. Certains de ces procédés reposent sur l'utilisation de solvants organiques et ne répondent donc pas entièrement au concept de chimie respectueuse de l'environnement.

Une autre voie consistant en la synthèse de dérivés *C*-glycosides comme nouvelle famille de tensioactifs est décrite dans la demande internationale WO 02/051828. Ces composés comportent un groupement méthylène (*C*-glycoside) à la place de l'atome d'oygène anomérique (O-glycoside). Le seul remplacement d'un atome d'oxygène anomérique par un atome de carbone *C*-anomérique peut entraîner des modifications des propriétés physico-chimiques, en particulier la valeur de la CMC (concentration micellaire critique), la tension interfaciale, l'hydrosolubilité, la balance hydrophile/lipophile, l'hydratation, les propriétés cristallines liquides, entre autres. Un inconvénient de certains procédés de synthèse proposés dans la demande WO 02/051828 tient à l'utilisation de co-solvants organiques, ce qui n'est pas suffisamment respectueux de l'environnement.

Il existe donc un besoin pour disposer de nouveaux composés tensioactifs ayant des performances et des tolérances améliorées.

Il existe également un besoin pour disposer de composés tensioactifs répondant à des normes environnementales telles que la biodégradabilité, l'éco-compatibilité.

Il existe encore un besoin pour disposer de nouveaux composés *C*-glycosides ayant un grand domaine d'application.

Il existe encore un besoin pour disposer de nouveaux procédés de préparation de composés *C*-glycosides qui soient davantage respectueux de l'environnement.

L'invention vise à répondre à tout ou partie de ces besoins.

### Composés C-glycosides de type alcools tertiaires

L'invention a pour objet, selon un premier de ses aspects, des composés *C*-glycoside présentant la formule (I) suivante : 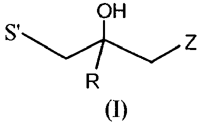 où:
S' représente un radical monosaccharidique, ou polysaccharidique présentant jusqu'à 20 unités sucre, sous forme pyranose et/ou furanose et de série L et/ou D;
la liaison S'-CH₂C(OH) représente une liaison de nature *C*-anomérique ;
R représente un radical alkyl linéaire ou ramifié, pouvant présenter une ou plusieurs insaturations, et comprenant 1 à 30 atomes de carbone, et ;
Z représente un radical éthylényl CH=CR1(R2) ou acétylényl C=CR3 tels que R1, R2 et R3 représentent indépendamment un hydrogène ou un radical alkyl linéaire ou ramifié, pouvant présenter une ou plusieurs insaturations et comprenant 1 à 30 atomes de carbone.

Les nouveaux composés *C*-glycosides de type alcool tertiaire de formule (1) présentent une grande stabilité chimique et/ou enzymatique, compatible avec un large domaine d'applications.

Par rapport notamment à des analogues dérivés O-glycosidiques ou à des analogues d'esters ou d'éthers dérivés de sucres, les dérivés *C*-glycosides conformes à l'invention sont susceptibles de présenter des propriétés avantageuses. En particulier, leur pouvoir solubilisant est susceptible d'être plus efficace et/ou plus spécifique en fonction des substrats. Leur pouvoir hydrotrope ou leur pouvoir de stabiliser des formulations (point de trouble) est également susceptible d'être meilleur. Leurs propriétés sensorielles peuvent être originales, en conférant notamment à des compositions cosmétiques un toucher riche, soyeux et non gras.

Compte tenu de la présence d'insaturations dans le radical Z et/ou de la présence d'un alcool tertaire, les composés selon l'invention sont susceptibles de présenter des propriétés physico-chimiques différentes de celles de l'art antérieur.

Préférentiellement, ledit radical S' est choisi dans le groupe constitué par les radicaux : glucosyl, galactosyl, mannosyl, xylosyl, lactosyl, N-acétyl-glucosaminosyl, N-acétylgalactosaminosyl.

En particulier, ledit radical S' est avantageusment choisi dans le groupe constitué par les radicaux : lactosyl, glucosyl, xylosyl.

Egalement préférentiellement, Z représente un radical CH=CH₂.

### Procédé A : Procédé de préparation de composés C-glycosides de formule (III)

L'invention a aussi pour objet, indépendamment ou en combinaison avec ce qui précède, un procédé de préparation de composés *C*-glycosides de formule (III) incluant une étape consistant à faire réagir dans un solvant aqueux ou en l'absence de solvant et en présence d'une base organique ou minérale B, un sucre S avec un phosphonate de formule (II), selon le schéma réactionnel suivant: 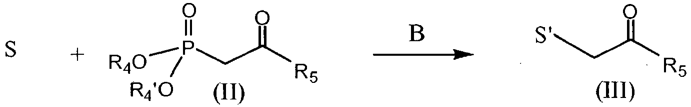 où:
S représente un monosaccharide ou un polysaccharide présentant jusqu'à 20 unités sucre, sous forme pyranose et/ou furanose et de série L et/ou D, ledit mono- ou polysaccharide présentant au moins une fonction hydroxyle libre ;
S' représente un radical monosaccharidique, ou polysaccharidique présentant jusqu'à 20 unités sucre, sous forme pyranose et/ou furanose et de série L et/ou D dérivé dudit monosaccharide ou polysaccharide S ;
la liaison S'-CH₂C(O) représente une liaison de nature *C*-anomérique ;
R4 et R4' représentent indépendamment un radical alkyl linéaire ou ramifié comprenant 1 à 6 atomes de carbone,
R5 représente un radical R, O-R, S-R ou NH-R où R représente un radical alkyl linéaire ou ramifié, pouvant présenter une ou plusieurs insaturations et comprenant 1 à 30 atomes de carbone.

On notera que dans le cadre de la présente description les monosaccharides ou polysaccharides S utilisés, outre une fonction hydroxyle libre, pourront par ailleurs présenter une ou plusieurs fonctions hydroxyles protégées ou non et/ou une ou plusieurs fonctions amines protégées ou non.

Selon une variante, B est préférentiellement une base minérale.

S peut être choisi dans le groupe constitué par: le glucose, le galactose, le mannose, le xylose, le lactose, la N-acétyl-glucosamine et la N-acétyl-galactosamine et les polysaccharides constitués d'unités sucre choisies parmi ceux-ci.

S peut en particulier comprendre du lactose et/ou du glucose et/ou du xylose.

Selon une variante R4 et R4' peuvent en particulier représenter chacun un radical méthyle CH₃.

Le procédé selon l'invention permet d'introduire une seule chaîne en position anomérique sans entrainer d'oligomérisation du sucre.

### Variante A1 en présence d'un solvant aqueux

Le procédé A1 peut, selon une variante A1, être mis en oeuvre dans un solvant aqueux, notamment dans de l'eau.

Dans ce cas, le procédé peut inclure les étapes consistant, notamment dans l'ordre suivant,
à dissoudre un équivalent dudit monosaccharide ou polysaccharide S dans une quantité suffisante d'eau ;
à additionner entre 1 et 5 équivalents dudit phosphonate de formule (II) et entre 1 et 10 équivalents de ladite base (B), à une température comprise entre 25°C et 100°C, sur une durée comprise entre 3 heures et 30 heures ;
à neutraliser le milieu réactionnel par un acide minéral ou organique ou par passage une résine acide ;
à concentrer puis co-évaporer avec un solvant organique choisi dans le groupe constitué par l'éthanol, le méthanol, le toluène et/ou l'isopropanol,
ou à sécher,
ou à lyophiliser.

Le procédé peut inclure de plus l'étape consistant à purifier le mélange réactionnel obtenu par chromatographie ou par cristallisation.

### Variante A2 sans solvant

Dans un autre mode de réalisation du procédé, le procédé est mis en oeuvre en l'absence de solvant.

Dans ce cas, le procédé peut inclure l'étape suivante :
dissoudre un équivalent dudit monosaccharide ou polysaccharide (S) et entre 1 et 10 équivalents de base (B) dans 3 à 25 équivalents de phosphonate de formule (II) à une température comprise entre 25°C et 100°C, notamment entre 50 et 70°C, sur une durée comprise entre 3 heures et 30 heures, notamment entre 20 et 25h..

Le procédé peut en outre inclure l'étape consistant à distiller le mélange réactionnel obtenu, le phosphonate alors récupéré pouvant être recyclé et engagé dans une nouvelle réaction.

Le procédé peut en outre inclure l'étape consistant à purifier le produit obtenu par chromatographie et/ou cristallisation.

### Procédé B : procédé de préparation de composés C-glycosides de formule (I) à partir de composés C-glycosides de formule (III')

L'invention a encore pour objet, selon un autre de ses aspects, un procédé de préparation de dérivés *C*-glycosides de type alcool tertiaire de formule (I) incluant une étape consistant à faire réagir un composé *C*-glycoside de formule (III') dans un solvant aqueux et en présence d'un métal, avec un composé halogéné de formule (IV) selon le schéma réactionnel suivant : 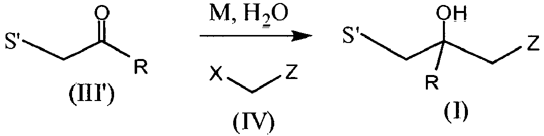 où:
S' représente un radical monosaccharidique, ou polysaccharidique présentant jusqu'à 20 unités sucre, sous forme pyranose et/ou furanose et de série L et/ou D dérivé dudit monosaccharide ou polysaccharide S ;
la liaison S'-CH₂C(O) représente une liaison de nature *C*-anomérique ;
R représente un radical alkyl linéaire ou ramifié, pouvant présenter une ou plusieurs insaturations, et comprenant 1 à 30 atomes de carbone, et ;
Z représente un radical éthylényl CH=CR1(R2) ou acétylényl C≡CR3 tels que R1, R2 et R3 représentent indépendamment un hydrogène ou un radical alkyl linéaire ou ramifié, pouvant présenter une ou plusieurs insaturations et comprenant 1 à 30 atomes de carbone ;
M représente un métal choisi parmi l'indium, le zinc ou le magnésium.

Le procédé peut ainsi inclure les étapes consistant à, notamment dans l'ordre suivant :
à dissoudre un équivalent dudit composé C-glycoside de formule (III') dans une quantité suffisante d'eau ;
à additionner entre 1 et 4 équivalents dudit composé halogéné de formule (IV) et entre 1 et 4 équivalents de métal (M) à une température comprise entre 20°C et 40°C sur une durée comprise entre 3 heures et 30 heures ; et,
à co-évaporer la phase aqueuse avec un solvant organique choisi dans le groupe constitué par l'éthanol, le méthanol, le toluène et/ou l'isopropanol,
ou à sécher,
ou à lyophiliser.

Le procédé peut en outre inclure l'étape consistant à laver le mélange réactionnel par un solvant organique tel que le dichlorométhane ou à centrifuger avec élimination du dépôt, après l'étape de dissolution.

Le procédé peut inclure l'étape consistant à purifier le mélange réactionnel obtenu par chromatographie et/ou cristallisation.

Le procédé peut conduire à l'obtention d'un mélange composé en pourcentage massique par rapport à la totalité du mélange, de 0 à 30% de composé *C*-glycoside de formule (III') et de 50 à 100% de dérivés *C*-glycosides de formule (I).

### Procédé C : procédé de préparation de composés C-glycosidzs de formule (I) à partir de sucre S

L'invention a encore pour objet, selon un autre de ses aspects, un procédé de préparation de dérivés *C*-glycosides de type alcools tertiaires de formule (I) consistant à faire réagir un sucre S avec un phosphonate de formule (II') en présence de solvant aqueux, d'un métal M, en présence d'une base B organique ou minérale, notamment minérale, et un composé halogéné de formule (IV), selon le schéma réactionnel suivant : 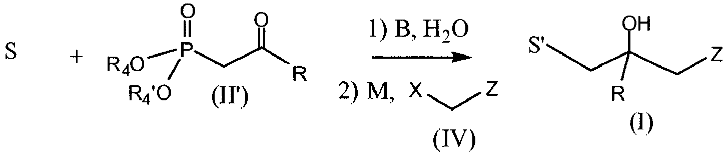 où:
S représente un monosaccharide ou un polysaccharide présentant jusqu'à 20 unités sucre, sous forme pyranose et/ou furanose et de série L et/ou D, ledit mono- ou polysaccharide présentant au moins une fonction hydroxyle libre ;
S' représente un radical monosaccharidique, ou polysaccharidique présentant jusqu'à 20 unités sucre, sous forme pyranose et/ou furanose et de série L et/ou D dérivé dudit monosaccharide ou polysaccharide S ;
la liaison S'-CH₂C(OH) représente une liaison de nature *C*-anomérique ;
R4 et R4' représentent indépendamment un radical alkyl linéaire ou ramifié comprenant 1 à 6 atomes de carbone,
R représente un radical alkyl linéaire ou ramifié, pouvant présenter une ou plusieurs insaturations, et comprenant 1 à 30 atomes de carbone, et ;
X représente un halogène,
Z représente une fonction éthylényl CH=CR1(R2) ou acétylényl C≡CR3 tel que R1, R2 et R3 représentent indépendamment un hydrogène ou un radical alkyl linéaire ou ramifié, pouvant présenter une ou plusieurs insaturations et comprenant 1 à 30 atomes de carbone, et
M représente un métal choisi parmi l'indium, le zinc ou le magnésium.

Le procédé selon l'invention peut inclure les étapes suivantes :
à dissoudre un équivalent dudit monosaccharide ou polysaccharide S dans une quantité suffisante d'eau,
à additionner entre 1 et 5 équivalents, notamment entre 2 et 3 équivalents, de phosphonate de formule (II'), et entre 1 et 10 équivalents, notamment entre 3 et 6 équivalents de base B, à une température comprise entre 25 et 100°C, notamment entre 50 et 70°C, sur une durée comprise entre 3 heures et 30 heures, préférentiellement entre 20 heures et 25 heures ;
à additionner entre 1 et 4 équivalents dudit composé halogéné de formule (IV) et entre 1 et 4 équivalents de métal (M) à une température comprise entre 20°C et 40°C sur une durée comprise entre 3 heures et 30 heures ; et,
à co-évaporer la phase aqueuse avec un solvant organique choisi dans le groupe constitué par l'éthanol, le méthanol, le toluène et/ou l'isopropanol,
ou à sécher,
ou à lyophiliser, préférentiellement à lyophiliser directement.

Le procédé peut en outre inclure, après l'étape d'addition, l'étape consistant à laver le mélange réactionnel par un solvant organique, notamment le dichlorométhane, ou à le centrifuger avec élimination du dépôt.

A la fin, le procédé peut inclure l'étape consistant à purifier le produit réactionnel obtenu par chromatographie et/ou cristallisation.

Le procédé peut conduire à l'obtention d'un mélange composé, en pourcentage massique par rapport à la totalité du mélange, de 0 à 20% de sucre S, de 0 à 30 % de composé *C*-glycoside de formule (III') et de 50 à 100% de dérivé *C*-glycoside de formule (I).

Selon un aspect de l'invention, le saccharide S utilisé dans le cadre du procédé selon celle-ci sera utilisé sous forme d'un jus de lactose. Un tel jus de lactose pourra par exemple provenir de l'industrie laitière.

### Utilisations

Les composés *C*-glycosides de formule (III) et les composés *C*-glycosides de formule (I) obtenus par les procédés A, B et C décrits plus haut sont susceptibles de présenter notamment des propriétés hydrotropes, solubilisantes, détergentes ou émulsifiantes et peuvent être ainsi utilisés dans des formulations pour des applications en détergence, en cosmétique, en pharmacie, dans les encres et les peintures, entre autres.

L'invention sera mieux comprise en référence à la description qui va suivre de différents exemples de modes de réalisation de celle-ci.

### Exemples

### Exemple 1 : Procédé A1 dans l'eau : Synthèse de la 1-(C-Lactosyl)-propan-2-one :

1 mmol de lactose et 9 mmol de K₂CO₃ (9 éq.) sont dissous dans 2 mL d'eau chauffée à 65°C. 3 mmol de diméthyl-2-oxopropylphosphonate (3 éq.) sont ajoutés. Le mélange réactionnel est laissé sous agitation environ 24 h à 65°C, puis il est neutralisé par ajout d'une solution d'acide chlorhydrique à 5%. Après lyophilisation, une purification par colonne de chromatographie sur gel de silice (Eluant : AcOEt/MeOH/H2O : 15/4/1) permet d'isoler le produit sous la forme d'un solide blanc avec un rendement de 67% (mélange β/α : 90/10). 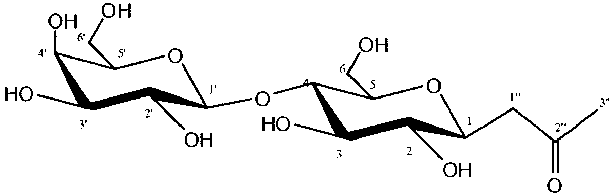
**RMN** : Produit majoritaire 1"-(β-*C*-Lactosyl)-propan-2"-one :
   ¹H (D₂O) (400 MHz): δ (ppm): 2.23 (s, 3H, H-3"), 2.68 (d, 1H, *J=* 9.52 Hz, *J*= 17.04 Hz, H-1"b), 2.99 (d, 1H, *J* = 2.88 Hz, *J* = 17.04 Hz, H-1 "a), 3.24 (t, 1H, *J* = 9.28 Hz, H-2), 3.51 (dd, 1H, H-5'), 3.52 (dd, 1H, H-2'), 3.59 (dd, 1H, H-6'b), 3.62 (dd, 1H, H-3), 3.67 (td, 1H, H-5), 3.70 (dd, 1H, H-6'a), 3.73 (dd, 1H, H-4), 3.74 (dd, 1H, H-6b), 3.75 (dd, 1H, H-3'), 3.78 (td, 1H, H-1), 3.85 (dd, 1H, H-4'), 3.88 (dd, 1H, H-6a), 4.39 (d, 1H, *J* = 7.72 Hz, H-1')
   ¹³C (D₂O) (100 MHz): δ (ppm): 30.14 (C-3"), 45.93 (C-1"), 60.35 (C-6), 61.35 (C-6'), 68.85 (C-4'), 71.26 (C- 2'), 72.81 (C-4), 73.06 (C-2), 75.35 (C-1), 75.65 (C-3'), 76.06 (C-3), 78.66 (C-5'), 78.68 (C-5), 103.21 (C-1'), 213.61 (C-2")
   [α]_{D}= +4.14° (c=1, MeOH)
**Spectroscopie Infra-Rouge** : (KBr) v (cm⁻¹) : 980-122 à (C-C, C-O strech), 1705 (C=O), 1945 (C-H), 3050-3600 (O-H strech)
**Spectrométrie de masse :**
   [M+Na]⁺ : m/z théorique = 405.13728 ; mesurée = 405.1373
   [M+K]⁺ : m/z théorique = 421.11122; mesurée = 421.1127

### Exemple 2 : Procédé A2 sans solvant : Synthèse de la 1-(C-Glucosyl)-heptan-2-one :

1 mmol de glucose et 3 mmol de K₂CO₃ (3 éq.) sont dissous dans 15 mmol de diméthyl-2-oxoheptylphosphonate (15 éq.) chauffé à 65°C. Le mélange réactionnel est laissé sous agitation environ 24 h à 65°C. Le phosphonate restant est ensuite distillé et peut être recyclé (70% du phosphonate engagé). Un mélange contenant le produit avec des résidus de phosphonates et de lactose est alors obtenu. Une purification par colonne de chromatographie sur gel de silice (Eluant : AcOEt/MeOH/H₂O : 15/4/1) permet d'isoler le produit sous la forme d'un solide jaune avec un rendement de 45% (mélange β/α : 70/30). 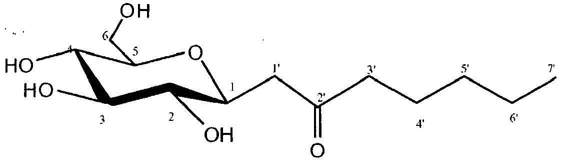
**RMN** : Produit majoritaire 1'-(β-*C*-glucosyl)-heptan-2'-one
   ¹H (CD₃OCD₃) (400 MHz): δ (ppm): 0.90 (3H, t, *J* = 7.2 Hz, H-7'), 1.29 (4H, m, H-5', H-6'), 1.51 (2H, q, *J* = 7.2 Hz, H-4'), 2.49 (3H, m, H3',H-1'a), 2.80 (1H, dd, *J* = 15.6, 2.8 Hz, H-1'b), 3.08 (1H, t, *J* = 8.8 Hz, H-2), 3.23 (1H, ddd, *J* = 9.2, 5.2, 2.8 Hz, H-5), 3.33 (1H, t, *J* = 8.6 Hz, H-4), 3.36 (1H, t, *J* = 8.6 Hz, H-3), 3.59 (1H, dd, *J* = 11.0, 5.2 Hz, H-6a), 3.68 (1H, td, *J* = 9.6, 3.2 Hz, H-1), 3.70 (1H, d, *J* = 10.8 Hz, H-6b)
   ¹³C (CD₃OCD₃) (100 MHz): δ (ppm): 14.99 (C-7'), 23.92 (C-6'), 24.60 (C-5'), 32.82 (C-4'), 44.34 (C-3'), 47.03 (C-1'), 63.79 (C-6), 72.74 (C-4), 75.69 (C-2), 77.75 (C-1), 80.38 (C-3), 81.82 (C-5), 209.73 (C-2')
   [α]_{D}= -15.9° (c=1, MeOH)
**Spectrométrie de masse** :
   [M+Na]⁺ : m/z théorique = 299.14706 ; mesurée = 299.1474

### Exemple 3 : Procédé A2 sans solvant : Synthèse de la 1-(C-Lactosyl)-tridecan-2-one :

1 mmol de lactose et 3 mmol de K₂CO₃ (3 éq.) sont dissous dans 15 mmol de diméthyl-2-oxotridecylphosphonate (15 éq.) chauffé à 75°C. Le mélange réactionnel est laissé sous agitation environ 24 h à 75°C. Le phosphonate restant est ensuite distillé et peut être recyclé (60% du phosphonate engagé). Un mélange contenant le produit avec des résidus de phosphonates et de lactose est alors obtenu. Une purification par colonne de chromatographie sur gel de silice (Eluant : AcOEt) permet d'isoler le produit sous la forme d'un solide blanc avec un rendement de 50% (mélange β/α : 55/45). 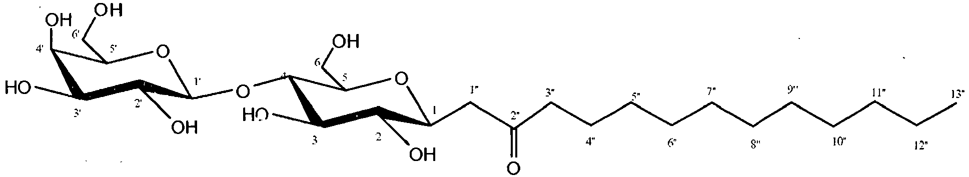
**RMN** : Produit majoritaire 1"-(β-*C*-lactosyl)-tridecan-2"-one:
   ¹H (CD₃OCD₃) (400 MHz): δ (ppm): 0.90 (3H, t, *J*= 6.8 Hz, H-13"), 1.20 (16H, m), 1.45 (2H, m, H-4"), 2.42 (2H, t, *J* = 5.6 Hz, H-3"), 2.51 (1H, dd, *J* = 16, 9.2 Hz, H-1"a), 2.77 (1H, t, *J* = 16,2.8 Hz, H-1"b), 3.06 (1H, t, *J* = 9.6 Hz, H-2), 3.47-3.73 (12H, m), 4.25 (1H, d, *J* = 7.2 Hz, H-1')
   ¹³C (CD₃OCD₃) (100 MHz): δ (ppm): 13.83 (C-13"), 22.89 (C-12"), 23.54 (C-4"), 28-32 (C5"-C11"), 42.76 (C-3"), 44,91 (C-1"), 61.05 et 61.48 (C-6 et C-6'), 68.83, 70.97, 71.11, 72.05, 73.23, 75.61, 76.19, 78.63, 79.32, 103.74 (C-1'), 212.16 (C-2")
**Spectrométrie de masse** :
   [M+Na]⁺ : m/z théorique = 545.29378 ; mesurée = 545.2941

### Exemple 4 : Procédé B

1 mmol de la 1-(*C*-Lactosyl)-propan-2-one obtenue selon l'exemple 1 est dissoute dans 2 mL d'eau, puis 1.5 mmol (1.5 éq.) de bromure d'allyle et 1.4 mmol (1.4 éq.) d'indium en poudre sont ajoutés. Le mélange est agité pendant 24 h à température ambiante.
Deux traitements sont alors possibles :
1) Un lavage au CH₂Cl₂ est effectué (4*7 mL). La phase aqueuse est concentrée à l'évaporateur rotatif puis laissée au lyophilisateur. Une colonne chromatographique sur gel de silice est ensuite effectuée (Eluant : AcOEt/MeOH/H₂O = 15/4/1), le produit est obtenu sous la forme d'un solide blanc avec un rendement de 85% (mélange β/α : 90/10).
2) Le mélange réactionnel est centrifugé. Après élimination du dépôt, la phase aqueuse est lyophilisée. Un mélange est alors obtenu de composition suivante : 15% 3-(*C*-Lactosyl)-2-propanone, 85% 5-(*C*-Lactosyl)-4-méthylpent-1-èn-4-ol.

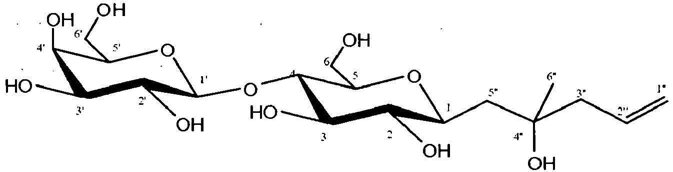

**RMN** : Produit majoritaire : 5"-(β-*C*-Lactosyl)-4"-méthylpent-1"-èn-4"-ol (Diastéréoisomères 1 et 2)
   ¹H (CD₃OD) (400 MHz): δ (ppm): 1.27 (s/s, 3H, H-6"1 et 2), 1.59 (dd, 1H, *Jgem* = 14.8 Hz, *J* = 9.28 Hz, H-5"), 1.98 (dd, 1H, *Jgem* =13.5 Hz, *J* = 9.96 Hz, H-5"a), 3.08 (t, 1H, *J* = 9.28 Hz, H-2), 2.25 (m, 2H, H-3" a et b), 3.26-3.38 (m, 2H), 3.43-3.60 (m, 4H), 3.66-3.88 (m, 3H), 4.32 (d/d, 1H, *J* = 7.72 Hz, H-1'1 et H-1'2), 5.04 (m, 2H, *J* = 14.84 Hz, H-1 "al/bl et H-1"a2/b2), 5.86 (m, 1H, *J* = 7.76Hz, H-2"1 et H-2"2)
   ¹³C (CD₃OD) (100 MHz): δ (ppm): 26.34 (C-6"), 42.57 (C-5"2), 43.14 (C-5"1), 47.44 (C-3"), 61.00 (C-6), 61.68 (C-6'), 69.33 (C-4'), 71.52 (C-2'), 73.73 (C-4), 74.40 (C-2), 76.91 (C-3'), 77.01 (C-1), 79.04 (C-3), 80.07 (C-5'), 80.10 (C-5), 104.09 (C-1'), 117.14 (C-1 "), 134.76 (C-2")
   [α]_{D}= +2.2° (c=1, MeOH)
**Spectroscopie Infra-Rouge** : (KBr) v (cm⁻¹) : 988-1216 (C-C, C-O strech), 1658 (C=C), 1935 (C-H), 3050-3600 (O-H strech)
**Spectrométrie de masse :**
   [M+Na]⁺ : m/z théorique = 447.18423; mesurée = 447.1839

### Exemple 5 : Procédé C : Synthèse directe du 5-(C-Glucosyl)-4-méthylpent-1-èn-4-ol :

1 mmol de glucose et 9 mmol de K₂CO₃ (9 éq.) sont dissous dans 2 mL d'eau chauffée à 65°C. 3 mmol de diméthyl-2-oxopropylphosphonate (3 éq.) sont ajoutés. Le mélange réactionnel est laissé sous agitation environ 24 h à 65°C, puis 1.5 mmol (1.5 éq.) de bromure d'allyle et 1.4 mmol (1.4 éq.) d'indium en poudre. Le mélange est agité pendant 24 h à température ambiante.
Deux traitements sont alors possibles :
1) Un lavage au CH₂Cl₂ est effectué (4*7 mL). La phase aqueuse est concentrée à l'évaporateur rotatif puis laissée au lyophilisateur. Une colonne chromatographique sur gel de silice est ensuite effectuée (Eluant : AcOEt/MeOH/H₂O = 15/4/1), le produit est obtenu sous la forme d'une huile incolore avec un rendement de 67% (mélange β/α: 90/10).
2) Le mélange réactionnel est centrifugé. Après élimination du dépôt, la phase aqueuse est lyophilisée. Un mélange est alors obtenu de composition suivante : 10% Glucose, 23% 3-(C-Glucosyl)-2-propanone, 67% 5-(C-Glucosyl)-4-méthylpent-1-èn-4-ol.

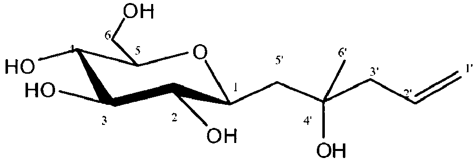

**RMN :** Produit majoritaire 5'-(β-*C*-Glucosyl)-4'-méthylpent-1'-èn-4'-ol (Diastéréoisomères 1 et 2)
   ¹H (CD₃OD) (400 MHz): δ (ppm): 1.29 (3H, s, H-6'), 1,69 (1H, m, H-5'b), 2.10 (1H, td, *J* =14.8,2.4 Hz, H-5'a), 2.37 (2H, m, H-3'a, H-3'b), 3.12 (1H, t, *J* = 8.8 Hz, H-2), 3.30-3.40 (3H, m, H-3, H-5, H-4), 3.54 (1H, tt, *J* = 8.8, 2.4 Hz, H-1), 3.64 (1H, m, H-6b), 3.91 (1H, dt, *J* = 12, 2.4 Hz, H-6a), 5.20 (1H, m, H-1'), 6.04 (1H, m, H-2')
   ¹³C (CD₃OD) (100 MHz): δ (ppm): 27.93 (C-6'), 44.27 (C-5'), 48.43 (C-3'), 63.51 (C-6), 72.41 (C-4), 76.10 (C-2), 78.66 (C-1), 80.05 (C-3), 81.89 (C-5), 118.60 (C-2'), 136.19 (C-1')
   [α]_{D}= +5.40° (c=1, MeOH)
**Spectroscopie Infra-Rouge :** (Nujol) ν (cm⁻¹) : 986-1215 (C-C, C-O strech), 1655 (C=C), 1940 (C-H), 3050-3600 (O-H strech)
**Spectrométrie de masse :**
   [M+Na] ⁺ : m/z théorique = 285.13141 ; mesurée = 285.1313

Des mesures tensiométriques, à savoir la mesure de la CMC (Concentration micellaire critique) et de la CMA (Concentration minimale d'agrégation) ont été effectuées sur différents composés obtenus selon l'invention.

Les résultats de ces mesures figurent dans le tableau 1 ci-dessous.

**Tableau 1**

| Composés *C*-glycoside | CMC*/CMA** (mM) | γ_{CMC/CMA}*** (mN.m⁻¹) |
|---|---|---|
| 5-*C*-(Glucosyl)-4-methylpent-1-èn-4-ol | 350 | 34 |
| 1-*C*-(Lactosyl)-heptan-2-one | 17 | 30 |
| 1-*C*-(Glucosyl)-heptan-2-one | 18 | 30 |
| 1-*C*-(Galactosyl)-heptan-2-one | 17.5 | 30 |
| 1-*C*-(Glucosyl)-tridecyl-2-one | 0.2 | 28 |

Les valeurs obtenues sont caractéristiques d'une bonne activité tensioactive. Selon les longueurs de chaîne, elles sont caractéristiques de composés possédant des propriétés hydrotropes, détergentes ou émulsifiantes.
* CMC : Concentration Micellaire Critique
**CMC: Concentration Minimale d'Agrégation
***γ_{CMC/CMA} : Tension interfaciale à la CMC/à la CMA

Le pouvoir solubilisant du 5-(β-*C*-Glucosyl)-4-méthylpent-1-èn-4-ol a également été évalué.

Pour tester le potentiel de solubilisation des produits, le colorant Disperse Red 13, dont la bande d'absorption maximale est à 525 nm, a été utilisé.

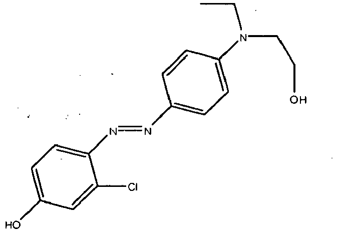

### Disperse Red DR13

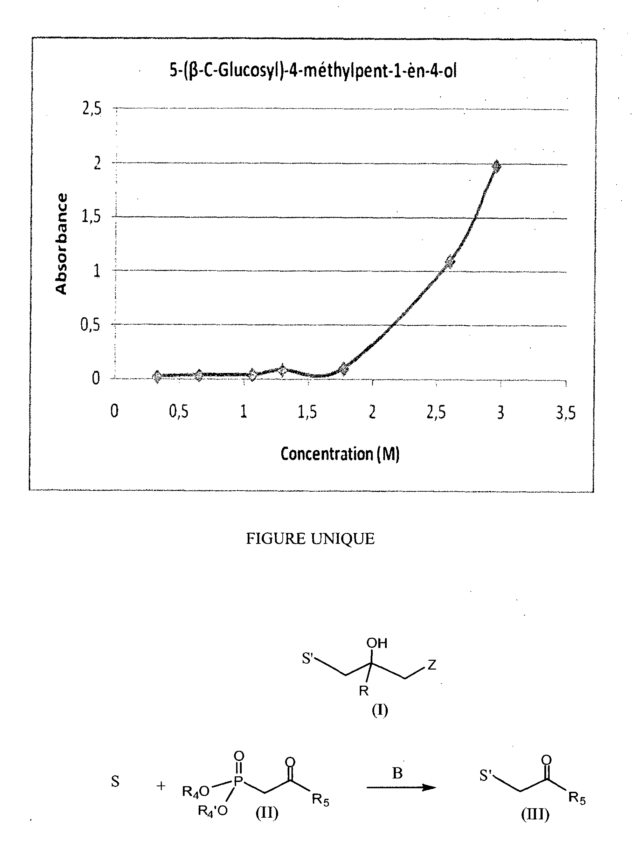

Différentes solutions de concentration décroissante en composé *C*-glycosidque 5-(β-*C-*Glucosyl)-4-méthylpent-1-èn-4-ol ont été préparées et une quantité suffisante de DR 13 (jusqu'à saturation de la solution) a été dissoute. L'absorbance des solutions obtenues a été mesurée par spectrométrie d'absorption UV à la longueur d'onde du colorant permettant ainsi de quantifier sa solubilisation. Les résultats de ces mesures figurent sur la figure 1.

Selon l'interprétation qui peut être faite de cette figure, le 5-(β-*C*-Glucosyl)-4-méthylpent-1-èn-4-ol présente un bon pouvoir solubilisant, car la quantité d'agent solubilisé est élevée à faible concentration d'agent solubilisant.

Enfin, les effets de différents composés *C*-glycosides obtenus selon l'invention sur le point de trouble d'un tensioactif ont été testés pour évaluer leur intérêt pour la stabilisation des formulations.

Le tensioactif pris en référence est le C6E2, le diéthylène glycol monohexylether. Les résultats obtenus figurent au tableau 2 ci-après.

**Tableau 2**

| Produit(s) (% mass.) | Point de trouble (°C) |
|---|---|
| C6E2 (1%) | 34 |
| C6E2 (1%) + 5-*C*-(Glucosyl)-4-methylpent-1-èn-4-ol (10%) | 44 |
| C6E2 (1%) + 5-*C*-(Galactosyl)-4-methylpent-1-èn-4-ol (10%) | 42 |
| C6E2 (1%)+ 5-*C*-(Lactosyl)-4-methylpent-1-èn-4-ol (10%) | 39 |
| C6E2 (1%) + 1-*C*-(Glucosyl)-heptan-2-one (1%) | 62 |
| C6E2 (1%) + 1-*C*-(Lactosyl)-heptan-2-one (1%) | 58 |

Une augmentation du point de trouble du tensioactif C6E2 est observée par l'ajout de 10% (chaîne méthylpent-1-èn-4-ol) ou 1% (chaîne hepta-2-one) de produit. Les produits selon l'invention permettent la stabilisation des formulations en fonction de la température et peuvent être utilisés comme co-tensioactifs.

Le 5-(β-*C*-Glucosyl)-4-méthylpent-1-èn-4-ol selon la présente invention a été mis en oeuvre dans les deux formulations suivantes.

| Formulation de composition d'un savon liquide | % en masse |
|---|---|
| 5-(β-*C*-Glucosyl)-4-méthylpent-1-èn-4-ol | 5 |
| PPG-14 palmeth-60 alkyl dicarbamate | 3 |
| PEG-200 | 3 |
| Ethanol | 3 |
| Decyl glucoside | 20 |
| EDTA | 0.2 |
| Triethanolamine | 0.5 |
| Eau | |
| | |

| Formulation de composition d'un détergent pour le nettoyage des surfaces lisses | % en masse |
|---|---|
| 5-(β-*C*-Glucosyl)-4-méthylpent-1-èn-4-ol | 2 |
| 1-*C*-(Glucosyl)-heptan-2-one | 3 |
| 1-*C*-(Glucosyl)-tridecan-2-one | 3 |
| Dodecyl-sulfate de sodium | 0.3 |
| Ethanol | 3 |
| Huile essentielle de citron 9286 (OSF) | 0.5 |
| Conservateur | 0.4 |
| | |
| Eau | |

## Revendications

1. Composé *C*-glycoside **caractérisé en ce qu'**il présente la formule (I) suivante : 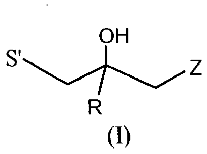 où:
S' représente un radical monosaccharidique, ou polysaccharidique présentant jusqu'à 20 unités sucre, sous forme pyranose et/ou furanose et de série L et/ou D ;
la liaison S'-CH₂C(OH) représente une liaison de nature *C*-anomérique ;
R représente un radical alkyl linéaire ou ramifié, pouvant présenter une ou plusieurs insaturations, et comprenant 1 à 30 atomes de carbone, et ;
Z représente un radical éthylényl CH=CR1(R2) ou acétylényl C≡CR3 tels que R1, R2 et R3 représentent indépendamment un hydrogène ou un radical alkyl linéaire ou ramifié, pouvant présenter une ou plusieurs insaturations et comprenant 1 à 30 atomes de carbone.

2. Composé selon la revendication 1, dans lequel ledit radical S' est choisi dans le groupe constitué par les radicaux : glucosyl, galactosyl, mannosyl, xylosyl, lactosyl, N-acétyl-glucosaminosyl, N-acétyl-galactosaminosyl.

3. Composé selon la revendication 2, dans lequel ledit radical S' est choisi dans le groupe constitué par les radicaux : lactosyl, glucosyl, xylosyl.

4. Composé selon l'une quelconque des revendications 1 à 3, **caractérisé en ce que** Z représente un radical CH=CH₂.

5. Procédé de préparation de composés *C*-glycosides de formule (III) **caractérisé en ce qu'**il inclut une étape consistant à faire réagir, dans un solvant aqueux ou en l'absence de solvant, et en présence d'une base organique ou minérale (B), un sucre (S) avec un phosphonate de formule (II), selon le schéma réactionnel suivant: 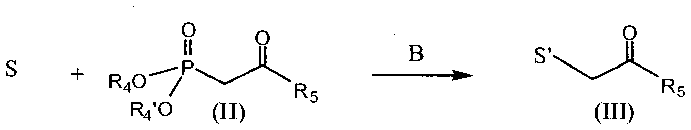 où:
S représente un monosaccharide ou un polysaccharide présentant jusqu'à 20 unités sucre, sous forme pyranose et/ou furanose et de série L et/ou D, ledit mono- ou polysaccharide présentant au moins une fonction hydroxyle libre ;
S' représente un radical monosaccharidique, ou polysaccharidique présentant jusqu'à 20 unités sucre, sous forme pyranose et/ou furanose et de série L et/ou D dérivé dudit monosaccharide ou polysaccharide S ;
la liaison S-CH₂C(O) représente une liaison de nature *C*-anomérique ;
R4 et R4' représentent indépendamment un radical alkyl linéaire ou ramifié comprenant 1 à 6 atomes de carbone ;
R5 représente un radical R, O-R, S-R ou NH-R où R représente un radical alkyl linéaire ou ramifié, pouvant présenter une ou plusieurs insaturations et comprenant 1 à 30 atomes de carbone.

6. Procédé selon la revendication 5, **caractérisé en ce que** S est choisi dans le groupe constitué par : le glucose, le galactose, le mannose, le xylose, le lactose, la N-acétyl-glucosamine ou la N-acétyl-galactosamine et les polysaccharides constitués d'unités sucre choisies parmi ceux-ci.

7. Procédé selon l'une quelconque des revendications 5 ou 6, dans lequel R4 et R4' représentent chacun un radical méthyl.

8. Procédé selon l'une quelconque des revendications 5 à 7, mis en oeuvre dans un solvant aqueux, notamment dans de l'eau.

9. Procédé selon la revendication 8, **caractérisé en ce qu'**il inclut les étapes consistant :
à dissoudre un équivalent dudit monosaccharide ou polysaccharide S dans une quantité suffisante d'eau ;
à additionner entre 1 et 5 équivalents dudit phosphonate de formule (II) et entre 1 et 10 équivalents de ladite base (B), à une température comprise entre 25°C et 100°C, sur une durée comprise entre 3 heures et 30 heures ;
à neutraliser le milieu réactionnel par un acide minéral ou organique ou par passage une résine acide ;
à concentrer puis co-évaporer avec un solvant organique choisi dans le groupe constitué par l'éthanol, le méthanol, le toluène et/ou l'isopropanol,
ou à sécher,
ou à lyophiliser.

10. Procédé selon l'une quelconque des revendications 5 à 7 **caractérisé en ce qu'**il est mis en oeuvre en l'absence de solvant.

11. Procédé selon la revendication 10 **caractérisé en ce qu'**il inclut l'étape suivante :
dissoudre un équivalent dudit monosaccharide ou polysaccharide (S) et entre 1 et 10 équivalents de base (B) dans 3 à 25 équivalents de phosphonate de formule (II) à une température comprise entre 25°C et 100°C sur une durée comprise entre 3 heures et 30 heures.

12. Procédé de préparation de composés C-glycosides de type alcool tertiaire de formule (I) incluant une étape consistant à faire réagir un composé C-glycoside de formule (III') dans un solvant aqueux et en présence d'un métal (M), avec un composé halogéné de formule (IV) selon le schéma réactionnel suivant, : 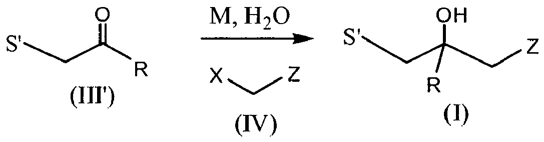 où
S' représente un radical monosaccharidique, ou polysaccharidique présentant jusqu'à 20 unités sucre, sous forme pyranose et/ou furanose et de série L et/ou D dérivé dudit monosaccharide ou polysaccharide S ;
la liaison S'-CH₂C(O) représente une liaison de nature *C*-anomérique ;
R représente un radical alkyl linéaire ou ramifié, pouvant présenter une ou plusieurs insaturations et comprenant 1 à 30 atomes de carbone ;
Z représente un radical éthylényl CH=CR1(R2) ou acétylényl C≡CR3 tels que R1, R2 et R3 représentent indépendamment un hydrogène ou un radical alkyl linéaire ou ramifié, pouvant présenter une ou plusieurs insaturations et comprenant 1 à 30 atomes de carbone ;
M représente un métal choisi parmi l'indium, le zinc ou le magnésium.

13. Procédé selon la revendication 12, **caractérisé en ce qu'**il comprend les étapes consistant :
à dissoudre un équivalent dudit composé C-glycoside de formule (III') dans une quantité suffisante d'eau ;
à additionner entre 1 et 4 équivalents dudit composé halogéné de formule (IV) et entre 1 et 4 équivalents de métal (M) à une température comprise entre 20°C et 40°C sur une durée comprise entre 3 heures et 30 heures ; et,
à co-évaporer la phase aqueuse avec un solvant organique choisi dans le groupe constitué par l'éthanol, le méthanol, le toluène et/ou l'isopropanol,
ou à sécher,
ou à lyophiliser.

14. Procédé de préparation de composés *C*-glycosides de type alcools tertiaires de formule (1) consistant à faire réagir un monosaccharide ou un polysaccharide S avec un phosphonate de formule (II') en présence d'eau, d'un métal (M) avec un composé halogéné de formule (IV), selon le schéma réactionnel suivant : 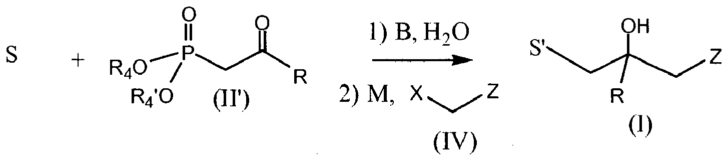 où:
S représente un monosaccharide ou un polysaccharide présentant jusqu'à 20 unités sucre, sous forme pyranose et/ou furanose et de série L et/ou D, ledit mono- ou polysaccharide présentant au moins une fonction hydroxyle libre ;
S' représente un radical monosaccharidique, ou polysaccharidique présentant jusqu'à 20 unités sucre, sous forme pyranose et/ou furanose et de série L et/ou D dérivé dudit monosaccharide ou polysaccharide S ;
la liaison S'-CH₂C(OH) représente une liaison de nature C-anomérique ;
R4 et R4' représentent indépendamment un radical alkyl linéaire ou ramifié comprenant 1 à 6 atomes de carbone ;
R représente un radical alkyl linéaire ou ramifié, pouvant présenter une ou plusieurs insaturations et comprenant 1 à 30 atomes de carbone ;
X représente un halogène ;
Z représente une fonction éthylényl CH=CR1(R2) ou acétylényl C≡CR3 tel que R1, R2 et R3 représentent indépendamment un hydrogène ou un radical alkyl linéaire ou ramifié, pouvant présenter une ou plusieurs insaturations et comprenant 1 à 30 atomes de carbone, et ;
M représente un métal choisi parmi l'indium, le zinc ou le magnésium.

15. Procédé selon la revendication 14, **caractérisé en ce qu'**il inclut les étapes consistant :
à dissoudre un équivalent dudit monosaccharide ou polysaccharide S dans une quantité suffisante d'eau ;
à additionner entre 1 et 5 équivalents, notamment entre 2 et 3 équivalents, de phosphonate de formule (II'), et entre 1 et 10 équivalents, notamment entre 3 et 6 équivalents de base B, à une température comprise entre 25 et 100°C, notamment entre 50 et 70°C, sur une durée comprise entre 3 heures et 30 heures, préférentiellement entre 20 heures et 25 heures ;
à additionner entre 1 et 4 équivalents dudit composé halogéné de formule (IV) et entre 1 et 4 équivalents de métal (M) à une température comprise entre 20°C et 40°C sur une durée comprise entre 3 heures et 30 heures ; et,
à co-évaporer la phase aqueuse avec un solvant organique choisi dans le groupe constitué par l'éthanol, le méthanol, le toluène et/ou l'isopropanol,
ou à sécher,
ou à lyophiliser, préférentiellement à lyophiliser directement.

16. Procédé selon l'une quelconque des revendications 5 à 15 **caractérisé en ce que** ledit saccharide S est utilisé sous la forme d'un jus de lactose.

## Patentansprüche

1. C-Glycosidverbindung, **dadurch gekennzeichnet, dass** sie die folgende Formel (I) aufweist : 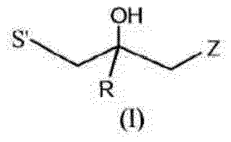 worin :
S' ein Monosaccharid- oder Polysaccharid-Radikal mit bis zu 20 Zuckereinheiten, in Pyranose- und/oder Furanose-Form und der L- und/oder D-Reihe darstellt;
die Bindung S'-CH₂C(OH) eine Bindung der C-anomeren Art darstellt;
R ein lineares oder verzweigtes Alkylradikal darstellt, das eine oder mehrere Unsättigung(en) aufweisen kann und 1 bis 30 Kohlenstoffatome umfasst; und Z ein Ethylenyl- CH=CR1(R2) oder Acetylenyl-Radikal C≡CR3 darstellt, welche derart sind, dass R1, R2 und R3 unabhängig einen Wasserstoff oder ein lineares oder verzweigtes Alkylradikal darstellen, das eine oder mehrere Unsättigung(en) aufweisen kann und 1 bis 30 Kohlenstoffatome umfasst.

2. Verbindung nach Anspruch 1, wobei das Radikal S' aus der Gruppe bestehend aus den Radikalen Glucosyl, Galactosyl, Mannosyl, Xylosyl, Lactosyl, N-Acetyl-Glucosaminosyl, N-Acetyl-Galactosaminosyl ausgewählt ist.

3. Verbindung nach Anspruch 2, wobei das Radikal S' aus der Gruppe bestehend aus den Radikalen Lactosyl, Glucosyl, Xylosyl ausgewählt ist.

4. Verbindung nach einem der Ansprüche 1 bis 3, **dadurch gekennzeichnet, dass** Z ein Radikal CH=CH₂ darstellt.

5. Verfahren zur Herstellung von C-Glycosidverbindungen der Formel (III), **dadurch gekennzeichnet, dass** es einen Schritt einschließt, der darin besteht, in einem wässrigen Lösungsmittel oder in Abwesenheit eines Lösungsmittels sowie in Gegenwart einer organischen oder mineralischen Base (B) einen Zucker (S) mit einem Phosphonat der Formel (II) nach dem folgenden Reaktionsschema reagieren zu lassen: 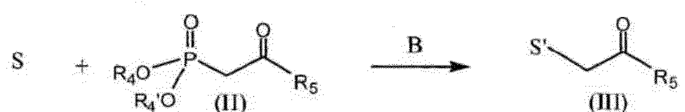 w or in
S ein Monosaccharid oder ein Polysaccharid mit bis zu 20 Zuckereinheiten, in Pyranose- und/oder Furanose-Form und der L- und/oder D-Reihe darstellt, wobei das Mono- oder Polysaccharid wenigstens eine freie Hydroxylfunktion aufweist;
S' ein Monosaccharid- oder Polysaccharid-Radikal mit bis zu 20 Zuckereinheiten, in Pyranose- und/oder Furanose-Form und der L- und/oder D-Reihe, abgeleitet aus dem Monosaccharid oder Polysaccharid S darstellt;
die Bindung S-CH₂C(O) eine Bindung der C-anomeren Art darstellt;
R4 und R4' unabhängig ein lineares oder verzweigtes Alkylradikal mit 1 bis 6 Kohlenstoffatom(en) darstellen;
R5 ein Radikal R, O-R, S-R oder NH-R darstellt, wobei R ein lineares oder verzweigtes Alkylradikal darstellt, das eine oder mehrere Unsättigung(en) aufweisen kann und 1 bis 30 Kohlenstoffatome umfasst.

6. Verfahren nach Anspruch 5, **dadurch gekennzeichnet, dass** S aus der Gruppe bestehend aus Glucose, Galactose, Mannose, Xylose, Lactose, N-Acetyl-Glucosamin oder N-Acetyl-Galactosamin und den Polysacchariden, die aus aus diesen ausgewählten Zuckereinheiten bestehen, ausgewählt ist.

7. Verfahren nach einem der Ansprüche 5 oder 6, wobei R4 und R4' jeweils ein Methylradikal darstellen.

8. Verfahren nach einem der Ansprüche 5 bis 7, das in einem wässrigen Lösungsmittel, insbesondere in Wasser durchgeführt wird.

9. Verfahren nach Anspruch 8, **dadurch gekennzeichnet, dass** es die Schritte einschließt, die darin bestehen:
ein Äquivalent des Monosaccharids oder Polysaccharids S in einer ausreichenden Menge Wasser zu lösen;
zwischen 1 und 5 Äquivalent(e) des Phosphonats der Formel (II) sowie zwischen 1 und 10 Äquivalent(e) der Base (B) bei einer Temperatur im Bereich zwischen 25 °C und 100 °C, über eine Dauer zwischen 3 Stunden und 30 Stunden zuzugeben;
das Reaktionsmedium durch eine mineralische oder organische Säure oder abschnittsweise ein Säureharz zu neutralisieren;
zu konzentrieren, anschließend mit einem organischen Lösungsmittel, das aus der Gruppe bestehend aus Ethanol, Methanol, Toluol und/oder Isopropanol ausgewählt ist, gemeinsam zu verdampfen,
oder zu trocknen,
oder zu lyophilisieren.

10. Verfahren nach einem der Ansprüche 5 bis 7, **dadurch gekennzeichnet, dass** es in Abwesenheit eines Lösungsmittels durchgeführt wird.

11. Verfahren nach Anspruch 10, **dadurch gekennzeichnet, dass** es den folgenden Schritt einschließt:
Lösen eines Äquivalents des Monosaccharids oder Polysaccharids (S) sowie 1 bis 10 Äquivalent(e) der Base (B) in 3 bis 25 Äquivalenten des Phosphonats der Formel (II) bei einer Temperatur im Bereich zwischen 25 °C und 100 °C, über eine Dauer zwischen 3 Stunden und 30 Stunden.

12. Verfahren zur Herstellung von C-Glycosidverbindungen vom Typ tertiärer Alkohol der Formel (I), das einen Schritt einschließt, der darin besteht, eine C-Glycosidverbindung der Formel (III`) in einem wässrigen Lösungsmittel und in Gegenwart eines Metalls (M) mit einer Halogenverbindung der Formel (IV) nach dem folgenden Reaktionsschema reagieren zu lassen: 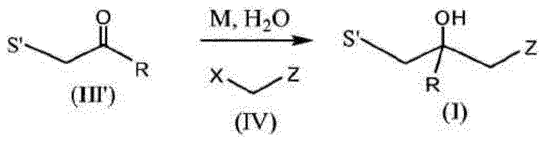 worin
S' ein Monosaccharid- oder Polysaccharid-Radikal mit bis zu 20 Zuckereinheiten, in Pyranose- und/oder Furanose-Form und der L- und/oder D-Reihe, abgeleitet aus dem Monosaccharid oder Polysaccharid S darstellt;
die Bindung S'-CH₂C(O) eine Bindung der C-anomeren Art darstellt;
R ein lineares oder verzweigtes Alkylradikal darstellt, das eine oder mehrere Unsättigung(en) aufweisen kann und 1 bis 30 Kohlenstoffatome umfasst;
Z ein Ethylenyl- CH=CR1(R2) oder Acetylenyl-Radikal C≡CR3 darstellt, welche derart sind, dass R1, R2 und R3 unabhängig einen Wasserstoff oder ein lineares oder verzweigtes Alkylradikal darstellen, das eine oder mehrere Unsättigung(en) aufweisen kann und 1 bis 30 Kohlenstoffatome umfasst;
M ein Metall ausgewählt aus Indium, Zink oder Magnesium darstellt.

13. Verfahren nach Anspruch 12, **dadurch gekennzeichnet, dass** es die Schritte umfasst, die darin bestehen:
ein Äquivalent der C-Glycosidverbindung der Formel (III') in einer ausreichenden Menge Wasser zu lösen;
zwischen 1 und 4 Äquivalent(e) der Halogenverbindung der Formel (IV) sowie zwischen 1 und 4 Äquivalent(e) des Metalls (M) bei einer Temperatur im Bereich zwischen 20 °C und 40 °C, über eine Dauer zwischen 3 Stunden und 30 Stunden zuzugeben;und
die wässrige Phase mit einem organischen Lösungsmittel, das aus der Gruppe bestehend aus Ethanol, Methanol, Toluol und/oder Isopropanol ausgewählt ist,
gemeinsam zu verdampfen,
oder zu trocknen,
oder zu lyophilisieren.

14. Verfahren zur Herstellung von *C*-Glycosidverbindungen vom Typ tertiäre Alkohole der Formel (I), das darin besteht, ein Monosaccharid oder ein Polysaccharid S mit einem Phosphonat der Formel (II`) in Gegenwart von Wasser, eines Metalls (M) mit einer Halogenverbindung der Formel (IV) nach dem folgenden Reaktionsschema reagieren zu lassen: 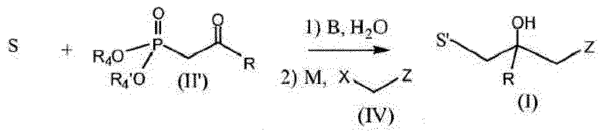 worin:
S ein Monosaccharid oder ein Polysaccharid mit bis zu 20 Zuckereinheiten, in Pyranose- und/oder Furanose-Form und der L- und/oder D-Reihe darstellt, wobei das Mono- oder Polysaccharid wenigstens eine freie Hydroxylfunktion aufweist;
S' ein Monosaccharid- oder Polysaccharid-Radikal mit bis zu 20 Zuckereinheiten, in Pyranose- und/oder Furanose-Form und der L- und/oder D-Reihe, abgeleitet aus dem Monosaccharid oder Polysaccharid S darstellt;
die Bindung S'-CH₂C(OH) eine Bindung der C-anomeren Art darstellt;
R4 und R4' unabhängig ein lineares oder verzweigtes Alkylradikal mit 1 bis 6 Kohlenstoffatom(en) darstellen;
R ein lineares oder verzweigtes Alkylradikal darstellt, das eine oder mehrere Unsättigung(en) aufweisen kann und 1 bis 30 Kohlenstoffatome umfasst;
X ein Halogen darstellt;
Z eine Ethylenyl- CH=CR1 (R2) oder Acetylenyl-Funktion C≡CR3 darstellt, derart,
dass R1, R2 und R3 unabhängig einen Wasserstoff oder ein lineares oder verzweigtes Alkylradikal darstellen, das eine oder mehrere Unsättigung(en) aufweisen kann und 1 bis 30 Kohlenstoffatome umfasst; und
M ein Metall ausgewählt aus Indium, Zink oder Magnesium darstellt.

15. Verfahren nach Anspruch 14, **dadurch gekennzeichnet, dass** es die Schritte einschließt, die darin bestehen:
ein Äquivalent des Monosaccharids oder Polysaccharids S in einer ausreichenden Menge Wasser zu lösen;
zwischen 1 und 5 Äquivalent(e), insbesondere zwischen 2 und 3 Äquivalente des Phosphonats der Formel (II`) sowie zwischen 1 und 10 Äquivalent(e), insbesondere zwischen 3 und 6 Äquivalente der Base B bei einer Temperatur im Bereich zwischen 25 und 100 °C, insbesondere zwischen 50 und 70 °C, über eine Dauer zwischen 3 Stunden und 30 Stunden, vorzugsweise zwischen 20 Stunden und 25 Stunden zuzugeben;
zwischen 1 und 4 Äquivalent(e) der Halogenverbindung der Formel (IV) sowie zwischen 1 und 4 Äquivalent(e) des Metalls (M) bei einer Temperatur im Bereich zwischen 20 °C und 40 °C, über eine Dauer zwischen 3 Stunden und 30 Stunden zuzugeben;und
die wässrige Phase mit einem organischen Lösungsmittel, das aus der Gruppe bestehend aus Ethanol, Methanol, Toluol und/oder Isopropanol ausgewählt ist,
gemeinsam zu verdampfen,
oder zu trocknen,
oder zu lyophilisieren, vorzugsweise direkt zu lyophilisieren.

16. Verfahren nach einem der Ansprüche 5 bis 15, **dadurch gekennzeichnet, dass** das Saccharid S in Form eines Lactosesaftes verwendet wird.

## Claims

1. *C*-glycoside compound, **characterized in that** it has the following formula (I) 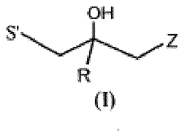 where:
S' represents a monosaccharide radical or a polysaccharide radical having up to 20 sugar units, in the pyranose and/or furanose form and of the L and/or D series;
the S'-CH₂C(OH) bond represents a bond of *C*-anomeric nature;
R represents a linear or branched alkyl radical which can have one or more unsaturated bonds and comprises 1 to 30 carbon atoms; and
Z represents an ethylenyl radical CH=CR1(R2) or acetylenyl radical C≡CR3, such that R1, R2 and R3 independently represent a hydrogen or a linear or branched alkyl radical which can have one or more unsaturated bonds and comprises 1 to 30 carbon atoms.

2. Compound according to claim 1, in which said radical S' is chosen from the group consisting of the radicals: glucosyl, galactosyl, mannosyl, xylosyl, lactosyl, N-acetylglucosaminosyl, N-acetylgalactosaminosyl.

3. Compound according to claim 2, in which said radical S' is chosen from the group consisting of the radicals: lactosyl, glucosyl, xylosyl.

4. Compound according to any one of claims 1 to 3, **characterized in that** Z represents a CH=CH₂ radical.

5. Process for the preparation of *C*-glycoside compounds of the formula (III), **characterized in that** it includes a stage consisting of reacting, in an aqueous solvent or in the absence of solvent, and in the presence of an organic or mineral base (B), a sugar (S) with a phosphonate of the formula (II) in accordance with the following reaction equation: 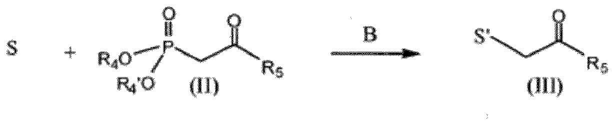 where:
S represents a monosaccharide or a polysaccharide having up to 20 sugar units, in the pyranose and/or furanose form and of the L and/or D series, said mono- or polysaccharide having at least one free hydroxyl function;
S' represents a monosaccharide radical or a polysaccharide radical having up to 20 sugar units, in the pyranose and/or furanose form and of the L and/or D series, derived from said monosaccharide or polysaccharide S;
the S-CH₂C(O) bond represents a bond of *C*-anomeric nature;
R4 and R4' independently represent a linear or branched alkyl radical comprising 1 to 6 carbon atoms;
R5 represents an R, O-R, S-R or NH-R radical, where R represents a linear or branched alkyl radical which can have one or more unsaturated bonds and comprises 1 to 30 carbon atoms.

6. Process according to claim 5, **characterized in that** S is chosen from the group consisting of: glucose, galactose, mannose, xylose, lactose, N-acetylglucosamine or N-acetylgalactosamine and the polysaccharides made up of sugar units chosen from these.

7. Process according to any one of claims 5 or 6, in which R4 and R4' each represent a methyl radical.

8. Process according to any one of claims 5 to 7, carried out in an aqueous solvent, in particular in water.

9. Process according to claim 8, **characterized in that** it includes the stages consisting of:
dissolving of one equivalent of said monosaccharide or polysaccharide S in a sufficient amount of water;
addition of between 1 and 5 equivalents of said phosphonate of the formula (II) and between 1 and 10 equivalents of said base (B) at a temperature of between 25°C and 100 °C over a period of between 3 hours and 30 hours;
neutralization of the reaction medium with a mineral or organic acid or by passage over an acid resin;
concentration and then co-evaporation with an organic solvent chosen from the group consisting of ethanol, methanol, toluene and/or isopropanol,
or drying,
or lyophilization.

10. Process according to any one of claims 5 to 7, **characterized in that** it is carried out in the absence of solvent.

11. Process according to claim 10, **characterized in that** it includes the following stage:
dissolving of one equivalent of said monosaccharide or polysaccharide (S) and between 1 and 10 equivalents of base (B) in 3 to 25 equivalents of phosphonate of the formula (II) at a temperature of between 25 °C and 100 °C over a period of between 3 hours and 30 hours.

12. Process for the preparation of *C*-glycoside compounds of the tertiary alcohol type of the formula (I), including a stage consisting of reacting a *C*-glycoside compound of the formula (III'), in an aqueous solvent and in the presence of a metal (M), with a halogenated compound of the formula (IV) in accordance with the following reaction equation: 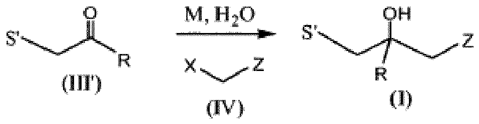 where
S' represents a monosaccharide radical or a polysaccharide radical having up to 20 sugar units, in the pyranose and/or furanose form of the L and/or D series, derived from said monosaccharide or polysaccharide S;
the S'-CH₂C(O) bond represents a bond of C-anomeric nature;
R represents a linear or branched alkyl radical which can have one or more unsaturated bonds and comprises 1 to 30 carbon atoms; and
Z represents an ethylenyl radical CH=CR1(R2) or acetylenyl radical C≡CR3, such that R1, R2 and R3 independently represent a hydrogen or a linear or branched alkyl radical, which can have one or more unsaturated bonds and comprises 1 to 30 carbon atoms;
M represents a metal chosen from indium, zinc or magnesium.

13. Process according to claim 12, **characterized in that** it comprises the stages consisting of:
dissolving of one equivalent of said C-glycoside compound of the formula (III') in a sufficient amount of water;
addition of between 1 and 4 equivalents of said halogenated compound of the formula (IV) and between 1 and 4 equivalents of metal (M) at a temperature of between 20 °C and 40 °C over a period of between 3 hours and 30 hours; and
co-evaporation of the aqueous phase with an organic solvent chosen from the group consisting of ethanol, methanol, toluene and/or isopropanol,
or drying,
or lyophilization.

14. Process for the preparation of *C*-glycoside compounds of the tertiary alcohol type of the formula (I), consisting of reacting a monosaccharide or a polysaccharide S with a phosphonate of the formula (II') in the presence of water and of a metal (M) with a halogenated compound of the formula (IV) in accordance with the following reaction equation: 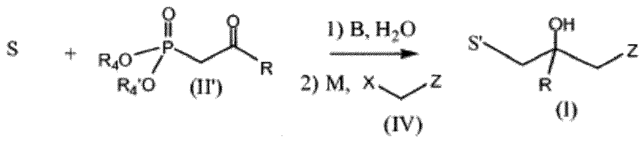 where:
S represents a monosaccharide or a polysaccharide having up to 20 sugar units, in the pyranose and/or furanose form and of the L and/or D series, said mono- or polysaccharide having at least one free hydroxyl function;
S' represents a monosaccharide radical or a polysaccharide radical having up to 20 sugar units, in the pyranose and/or furanose form and of the L and/or D series, derived from said monosaccharide or polysaccharide S;
the S-CH₂C(OH) bond represents a bond of *C*-anomeric nature;
R4 and R4' independently represent a linear or branched alkyl radical comprising 1 to 6 carbon atoms;
R represents a linear or branched alkyl radical which can have one or more unsaturated bonds and comprises 1 to 30 carbon atoms;
X represents a halogen;
Z represents an ethylenyl radical CH=CR1(R2) or acetylenyl radical C≡CR3, such that R1, R2 and R3 independently represent a hydrogen or a linear or branched alkyl radical, which can have one or more unsaturated bonds and comprises 1 to 30 carbon atoms; and
M represents a metal chosen from indium, zinc or magnesium.

15. Process according to claim 14, **characterized in that** it includes the stages consisting of:
dissolving of one equivalent of said monosaccharide or polysaccharide S in a sufficient amount of water;
addition of between 1 and 5 equivalents, in particular between 2 and 3 equivalents of phosphonate of the formula (II') and between 1 and 10 equivalents, in particular between 3 and 6 equivalents of base B, at a temperature of between 25 and 100 °C, in particular between 50 and 70 °C, over a period of between 3 hours and 30 hours, preferably between 20 hours and 25 hours;
addition of between 1 and 4 equivalents of said halogenated compound of the formula (IV) and between 1 and 4 equivalents of metal (M) at a temperature of between 20 °C and 40 °C over a period of between 3 hours and 30 hours; and
co-evaporation of the aqueous phase with an organic solvent chosen from the group consisting of ethanol, methanol, toluene and/or isopropanol,
or drying,
or lyophilization, preferably direct lyophilization.

16. Process according to any one of claims 5 to 15, **characterized in that** said saccharide S is used in the form of a lactose juice.
